# EUROPÄISCHE PATENTSCHRIFT

(11) **EP 0 908 442 B1**
(45) Veröffentlichungstag und Bekanntmachung des Hinweises auf die Patenterteilung: **24.04.2002**
(21) Anmeldenummer: 98118579.6
(22) Anmeldetag: 01.10.1998
(51) Int. Cl.: C07C 201/08, C07C 205/06

(54) **Verfahren zur Herstellung von Dinitrotoluol in adiabatischer Fahrweise**
Process for the preparation of dinitrotoluene under adiabatic conditions
Procédé pour la préparation de dinitrotoluène dans des conditions adiabatiques

(30) Priorität: 13.10.1997 DE 19745119
(43) Veröffentlichungstag der Anmeldung: 14.04.1999
(73) Patentinhaber: BAYER AG, 51368 Leverkusen (DE)
(72) Erfinder: Klingler, Uwe, 41468 Neuss (DE); Pirkl, Hans-Georg, Dr., 51061 Köln (DE); Schieb, Thomas, Dr., 51503 Rösrath (DE); Wastian, Dietmar, 41542 Dormagen (DE)

(56) Entgegenhaltungen:
- EP-A- 0 597 361
- EP-A- 0 696 569
- EP-A- 0 696 570
- US-A- 5 345 012

## Beschreibung

Die vorliegende Erfindung betrifft ein Verfahren zur Herstellung von Dinitrotoluol (DNT) durch Nitrierung von Toluol mit Nitriersäure unter adiabatischen Bedingungen.

Es ist bekannt, Toluol auf adiabatischem Wege unter Bildung von Dinitrotoluol zu nitrieren. Hierzu wird Toluol mit Nitriersäure, einem Gemisch aus Schwefel- und Salpetersäure, welches einen HNO₃-Gehalt von 1 bis 20 Gew.% besitzt, unter guter Durchmischung zur Reaktion gebracht. Das heiße Reaktionsprodukt wird einer Phasentrennung und einer Ausdampfung von Wasser unterworfen, wonach die aufkonzentrierte Absäure wieder in den Prozeß zurückgeführt wird. Bei der Aufkonzentrierung der Absäure wird das bei der Nitrierung gebildete Reaktionswasser und gegebenenfalls weiteres Wasser ausgetrieben. Zur Wasseraustreibung wird die Reaktionswärme genutzt.

Hierbei ergibt sich die Schwierigkeit, daß mit dem abgetrennten Wasser ein Teil des in der Säure noch gelösten DNT mit übergeht. Das mit dem Wasserdampf übergetretene DNT wird unter den Kondensationsbedingungen fest und belegt die Kühlflächen des Kondensations-Wärmetauschers. Der Festpunkt eines typischerweise hergestellten DNT-Isomerengemisches beträgt ca. 55°C. Durch die Wandbelagsbildung wird der Wärmeübergang wesentlich verschlechtert, der Wasserdampf kann nicht mehr in ausreichendem Maße kondensiert werden und der Kondensator muß wiederholt außer Betrieb genommen und gereinigt werden.

Beim konventionellen, "isothermen" zweistufigen Verfahren zur Herstellung von DNT behilft man sich dadurch, daß man Mononitrotoluol (MNT), welches im ersten Nitrierschritt gebildet und isoliert wird, in den Brüdenraum des Verdampfers einspritzt (DE-A-3 409 719). Das so eingespritzte MNT bewirkt eine Schmelzpunkterniedrigung des DNTs und sorgt somit dafür, daß die Brüden auch unter den Kondensationsbedingungen des Wassers flüssig bleiben. Die über Phasentrennung isolierte organische Phase des Brüdenkondensates wird in die Reaktoren der Dinitrierstufe zurückgeführt. Diese Lösung kann beim Verfahren der einstufigen, adiabatischen Dinitrierung von Toluol (EP-A-597 361) nicht zur Anwendung gelangen, da im Prozeß kein isolierbarer MNT-Strom vorliegt. MNT wird beim adiabaten, einstufigen Verfahren zwar als Zwischenprodukt gebildet, aber sofort zu DNT weiternitriert.

EP-696 569 beschreibt eine Verfahrensvariante für die einstufig-adiabatische DNT-Herstellung, die das angesprochene Problem löst. Die Nitrierung wird hierbei so durchgeführt, daß noch geringe Mengen an Mononitrotoluol nach der Nitrierung in der Reaktionsmischung vorliegen. Die Aufkonzentrierung der Absäure der Reaktion wird vor der Abtrennung der organischen Bestandteile durchgeführt. Das den Reaktor verlassende, noch MNT-haltige Reaktionsprodukt tritt direkt in die Aufkonzentrierung ein, wobei das noch vorhandene MNT bevorzugt mit Wasser und DNT-Anteilen über Kopf abgedampft wird. Die nach der Reaktion im Produktgemisch verbleibende MNT-Menge muß hierbei so gewählt werden, daß es im Aufkonzentrierschritt zu keiner Belegung des Kondensations-Wärmetauschers durch organische Produkte kommt.

Nachteilig bei dem in EP-696 569 beschriebenen Verfahren ist, daß das MNT in der Aufkonzentrierung nicht vollständig mit dem Wasser verdampft und immer gewisse MNT-Reste im Reaktionsprodukt verbleiben. Dieses MNT stellt einen Ausbeuteverlust dar, da es nicht vom DNT abgetrennt und in die Nitrierung zurückgeführt wird. Die Abtrennung erfolgt konventionell erst nach der Hydrierung des DNTs zum TDA, wo das aus dem MNT gebildete Toluylinamin destillativ abgetrennt wird. Dies bedeutet für das Hydrierverfahren einen zusätzlichen Destillationsaufwand sowie Zusatzkosten für Wasserstoff, Katalysator und Energie.

Aus den oben angeführten Gründen wird man bestrebt sein, den MNT-Gehalt im Reaktionsprodukt möglichst niedrig einzustellen, um nur mit der gerade zum Freihalten des Brüdenkondensators notwendigen minimalen Menge an MNT zu arbeiten.

Im praktischen Betrieb ist es jedoch oft problematisch - in der Praxis sogar zumeist unmöglich - derartige Grenzzustände einzuhalten. Darüber hinaus limitiert die Notwendigkeit eines Mindest-MNT-Restgehaltes die erzielbare DNT-Produktreinheit.

Außerdem stellt eine derartige Prozeßführung - problemlose Brüdenkondensation nebst möglichst MNT-freiem DNT - sehr hohe Anforderungen an die Verdampfungseinheit. In der Praxis dürfte es kaum möglich sein, eine Verdampfungseinheit zu konstruieren und zu betreiben, die beide Kriterien erfüllt. Ein gewisser Restgehalt an MNT im Nitroprodukt und damit ein Ausbeuteverlust wird letztendlich unvermeidlich sein. Weiterhin ist es äußerst schwierig, durch die Prozeßführung einen so engen Schwankungsbereich des MNT-Restgehaltes am Reaktorablauf zu garantieren, damit die Brüdenkondensation im vorgenannten Sinne funktioniert und nicht durch Feststoffbildung versagt.

Es bestand somit die Aufgabe, ein kontinuierliches, adiabatisches Verfahren zur Herstellung von Dinitrotoluol zur Verfügung zu stellen, bei dem die bisher auftretenden Probleme mit festen Ablagerungen im Kondensationsschritt der Brüden sicher vermieden werden und das wirtschaftlich und technisch in einfacher Weise zu betreiben ist.

Gegenstand der Erfindung ist daher ein Verfahren zur Herstellung von Dinitrotoluolen durch adiabatische Nitrierung von Toluol mit Nitriersäure bei Temperaturen von 60 bis 200°C und einem Molverhältnis von Toluol zu Salpetersäure von 1:1,5 bis 1:3,0, das dadurch gekennzeichnet ist, daß man das erhaltene Reaktionsgemisch bis zu einem Wassergehalt von bis zu 30 Gew.% aufkonzentriert, das im Reaktionsgemisch vorhandene Dinitrotoluol vor oder nach der Aufkonzentrierung aus dem Reaktionsgemisch ganz oder teilweise entfernt, das in den Brüden, die bei der Aufkonzentrierung des Reaktionsgemisches erhalten werden, noch vorhandene DNT durch Zugabe eines Lösungsmittels flüssig hält, das den Brüden zugegebene Lösungsmittel zusammen mit dem DNT von der wäßrigen Phase separiert, anschließend entweder das DNT von dem Lösungsmittel trennt, das Lösungsmittel wieder den Brüden zuführt und das aus den Brüden abgetrennte DNT entweder mit dem aus dem Reaktionsgemisch erhaltenen DNT vereinigt oder wieder in die Reaktion zurückführt oder das zusammen mit dem Lösungsmittel separierte DNT direkt in die Reaktion wieder zurückführt. Das erfindungsgemäße Verfahren wird bevorzugt bei Temperaturen von 90 bis 180°C, insbesondere 95 bis 170°C und ganz besonders bevorzugt bei 100 bis 160°C durchgeführt.

Das Molverhältnis von Toluol zu Salpetersäure beträgt beim erfindungsgemäßen Verfahren bevorzugt 1:1,7 bis 1:2,5, besonders bevorzugt 1:1,8 bis 1:2,2.

Als Nitriersäure wird bei dem erfindungsgemäßen Verfahren eine üblicherweise bei Nitrierung von Aromaten verwendete Nitriersäure eingesetzt. Die Nitriersäure, ein Gemisch aus Schwefel- und Salpetersäure, besitzt im allgemeinen einen Salpetersäuregehalt von 0,5 bis 15, bevorzugt 1,5 bis 8 Gew.-%.

Als Lösungsmittel, die den Brüden zugegeben werden, kommen prinzipiell alle organischen Stoffe in Frage, die DNT lösen und eine Bildung von Belägen auf Wärmetauschern verhindern können. Als Lösungsmittel kommen daher insbesondere solche organische Verbindungen in Frage, die bei Normaldruck einen Siedepunkt im Bereich von 80 bis 250°C, bevorzugt 100 bis 200°C besitzen. Hierzu zählen vor allem C₁-C₁₅-Kohlenwasserstoffe, die auch substituiert sein können, beispielsweise durch Nitrogruppen oder Halogene. Darüber hinaus eignen sich als Lösungsmittel auch gegebenenfalls durch Halogene substituierte Aromaten und Olefine sowie Benzine. Beispielsweise können als Lösungsmittel verwendet werden Toluol, Xylol, Chlorbenzol, Dichlorbenzol, Trichlorbenzol, Isododecan, Dodecan sowie die Mononitrotoluole. Besonders bevorzugt werden als Lösungsmittel Toluol und/oder die Mononitrotoluole als prozeßeigene Stoffe eingesetzt. Selbstverständlich können die Lösungsmittel einzeln oder im Gemisch untereinander verwendet werden.

Die Lösungsmittel werden den Brüden bzw. dem Brüdenkondensat in einer solchen Menge zugegeben, so daß ein Gewichtsverhältnis von Lösungsmittel zu in den Brüden vorhandenem DNT von 50:1 bis 1:10, bevorzugt 20:1 bis 1:5, vorliegt. Für das Gelingen des erfindungsgemäßen Verfahrens ist es von entscheidender Bedeutung, daß den Brüden die oben erwähnten Lösungsmittel separat zugegeben werden und daß die Gewichtsverhältnisse von Lösungsmittel zu DNT in den Brüden in den zuvor erwähnten Bereichen liegen.

Wie zuvor erwähnt, wird das bei der Reaktion erhaltene Reaktionsgemisch bis zu einem Wassergehalt von bis zu 30 Gew.-% aufkonzentriert. Bevorzugt wird das Reaktionsgemisch bis zu einem Wassergehalt von bis zu 27 Gew.-%, besonders bevorzugt bis zu einem Wassergehalt von maximal 23 Gew.-% aufkonzentriert.

Das in den Brüden bzw. Brüdenkondensaten, die bei der Aufkonzentrierung des Reaktionsgemisches erhalten werden, noch vorhandene DNT liegt in einem Mengenbereich von üblicherweise 2 bis 50 Gew.-%, bezogen auf die gesamte Brüdenmenge.

Die Aufkonzentrierung des Reaktionsgemisches kann in üblicher Weise durchgeführt werden, beispielsweise durch Destillation oder Flashen. Das nach der Abtrennung von DNT aufkonzentrierte Reaktionsgemisch, im wesentlichen bestehend aus einer wäßrigen Phase aus aufkonzentrierter Schwefelsäure und gegebenenfalls noch restlichen organischen Bestandteilen, wie Dinitrotoluol, Mononitrotoluol, Nitrokresole, Nitrobenzoesäure oder Nitrosylschwefelsäure kann wieder in die Reaktion zurückgeführt werden.

Die Ausbeute des nach dem erfindungsgemäßen Verfahren hergestellten DNTs beträgt nach der konventionellen Roh-DNT-Reinigung ca. ≥96 %; die Reinheit des erhaltenen DNTs liegt bei ≥98 %.

### Beispiele

### Beispiel 1: Brüdenkondensation mit MNT-Einspritzung als Lösungsmittel

Der Reaktor ist ein Rohrreaktor, bestehend aus einem unteren Teil (ca. 1m lang Durchmesser 28 mm) und einem oberen Teil mit 8 m Länge und einem Durchmesser von 80 mm. Die Reaktanden werden mittels einer Düse miteinander intensiv in Kontakt gebracht und vermischt. Mit 30 eingebauten Siebböden wird die erzeugte Dispersion entlang der Reaktorlängsachse aufrechterhalten.

Dem kontinuierlich betriebenen adiabaten Reaktor werden folgende Eingangsströme bei geändertem Gesamtumsatz zugeführt.:

| | | |
|---|---|---|
| Toluol | = 55,1 kg/h | (A) |
| 68,5 % HNO₃ | = 114,6 kg/h | (B) |
| 80,5 % H₂SO₄ | = 2002,1 kg/h | (C) |

Toluol (A) und Salpetersäure (B) werden bei einer Umgebungstemperatur von ca. 20°C zudosiert. Die Kreislaufschwefelsäure (C) wird mit einer Temperatur von ca. 110°C in den Reaktor geführt. Das am Reaktorende austretende 149°C heiße Reaktionsgemisch bestehend aus 78 gew.-%iger Kreislaufschwefelsäure, 89,3 kg/h Dinitrotoluol und 10,2 kg/h Mononitrotoluol wird in der Entspannungsverdampfung bei 30 mbar aufkonzentriert. Das Reaktionsgemisch kühlt sich durch Entzug der Verdampfungswärme auf 110°C ab. Die Temperatur von 110°C im Flash-Verdampfer wird durch zusätzliche Wärmezufuhr gehalten. Mit den Wasserdampfbrüden werden ca. 9,9 kg/h DNT und 5,7 kg/h MNT mitgerissen. Auf den Brüdenkondensator wurden zusätzlich 39,9 kg/h MNT am Kopf aufgegeben. Unter den vorliegenden Bedingungen konnten keine Wandanbackungen von DNT am Brüdenkondensator, der bei einer Kondensationstemperatur von 25°C betrieben wird, beobachtet werden. Im Phasentrenner und im Flash beobachtet man auch eine gewisse Weiternitrierung von MNT zu DNT, was nach der Phasentrennung ein isoliertes DNT mit 0,08 % MNT ergibt.

### Beispiel 2: Brüdenkondensation mit Toluol-Einspritzung als Lösungsmittel

Als Laborreaktor stand ein Düsenrohrreaktor zur Verfügung. Die Eduktströme bestanden jeweils aus reinem Toluol und einer Mischung von wäßriger Salpetersäure mit wäßriger Schwefelsäure, sogenannter Mischsäure. Die Eduktströme wurden auf 115°C thermostatisiert und kontinuierlich in den Reaktor dosiert. Der Düsenrohrreaktor wurde isoliert, um einen Temperaturverlust bei adiabatischem Betrieb zu vermeiden. Die Zusammensetzung der Mischsäure wurde gravimetrisch hergestellt und besteht aus 31.053 g H₂SO₄, 1.553 g HNO₃ und 9.358 g H₂O. Mischsäure strömte mit einem Volumenstrom von vier Litern/Stunde, das stöchiometrische Verhältnis von HNO₃ zu Toluol wurde auf 2,14 eingestellt. Zum Ausgleich von etwaigen Wärmeverlusten wurde am Reaktorende mit einem Ölthermostaten nachgeheizt. Das mit 160°C den Reaktor verlassende Reaktionsgemisch wurde bei einem Vakuum von 70 mbar einer Flash-Verdampfung unterworfen. In einem Kondensator mit 12°C Kühlwassertemperatur wurden die Brüden niedergeschlagen. In die Brüdenleitung, unmittelbar vor den Kondensator, wurden innerhalb von sieben Stunden 961 g Toluol gleichmäßig eingespritzt. Die Brüden sind flüssig ohne feste Anbackungen an den kalten Kühlerflächen abgelaufen. Das erhaltene Brüdenkondensat wurde in eine wäßrige und eine organische Phase getrennt und in seiner Zusammensetzung analysiert.

Die den Flash-Verdampfer flüssig verlassende Mischung aus aufkonzentrierter Schwefelsäure und Roh-DNT wurde bei 120°C in eine wäßrige (41.236 g) und eine organische Phase (660,2 g) getrennt. Die abgetrennte wäßrige Phase enthielt 1,39 Gew.% DNT und 78,5 Gew.% H₂SO₄. Der Rest bestand im wesentlichen aus Wasser.

Es wurden die folgenden Produktverteilungen im organischen Brüdenkondensat und im flüssigen, organischen Flash-Verdampferablauf (nach Abtrennung und Wäsche) ermittelt (MNT = Mononitrotoluol, DNT = Dinitrotoluol; Angaben in GC-Flächen%, Detektor: FID, Trennsäule: OV 1701):

| | **ORGANISCHES BRÜDENKONDENSAT** | **ORGANISCHER FLASH- ABLAUF** |
|---|---|---|
| Toluol | 7,10 | 0 |
| 2-MNT | 1,14 | 0 |
| 3-MNT | 0,92 | 0 |
| 4-MNT | 3,70 | 0 |
| 2,6-DNT | 32,85 | 27,65 |
| 2,5-DNT | 1,27 | 2,04 |
| 2,4-DNT | 49,27 | 63,34 |
| 2,3-DNT | 2,38 | 3,02 |
| 3,4-DNT | 1,36 | 3,94 |

### Beispiel 3: Brüdenkondensation mit Toluol-Einspritzung als Lösungsmittel und Rückführung in den Reaktor

Der Reaktor ist ein Rohrreaktor, bestehend aus einem unteren Teil ca. 1m lang mit Durchmesser 28 mm und einem oberen Teil mit 8 m Länge und einem Durchmesser von 80 mm. Die Reaktanden werden mittels einer Düse miteinander intensiv in Kontakt gebracht und vermischt. Mit 30 eingebauten Siebböden wird die erzeugte Dispersion entlang der Reaktorlängsachse aufrechterhalten.

Im kontinuierlichen Betrieb werden dem adiabatisch arbeitenden Reaktor folgende Eingangsströme zugeführt:

| | |
|---|---|
| Toluol | 55,0 kg/h |
| HNO₃ 65% | 119,0 kg/h |
| H₂SO₄ 78,8 % | 1647,7 kg/h |

Toluol und Salpetersäure werden bei einer Umgebungstemperatur von ca. 20°C zudosiert, wobei direkt vor dem Reaktor nur 27,5 kg/h Toluol dosiert werden. Die Kreislaufschwefelsäure wird mit einer Temperatur von 115°C in den Reaktor geführt. Das am Reaktorende austretende 155°C heiße Reaktionsgemisch wird der Aufkonzentriereinheit zugeführt und dort bei einem Druck von 78 mbar eingedampft. Das Reaktionsgemisch kühlt sich durch Entzug der Verdampfungswärme auf 132°C ab. Die Temperatur in der Aufkonzentriereinheit wird durch zusätzliche, indirekte Wärmezufuhr gehalten. Mit den Wasserdampfbrüden werden 12,8 kg/h DNT und 1,8 kg/h MNT mitgerissen. Auf den Brüdenkondensator werden 27,5 kg/h Toluol am Kopf aufgegeben. Unter diesen Bedingungen werden keine Ablagerungen an der Kondensationseinheit beobachtet, welche bei 25°C betrieben wird. Die kondensierten Brüden werden einer Phasentrennung zugeführt, wo die organische Phase von der anorganischen Phase abgetrennt und in den Reaktor zurückgeführt wird. Der Sumpf aus der Aufkonzentrierung tritt über einen barometrischen Verschluß im Normaldruckbereich aus, wird auf 115°C abgekühlt und einer Phasentrennung zugeführt. Hier werden 108 kg/h organische Phase abgetrennt, die zu 99,7 % aus DNT und zu 0,03 % aus MNT bestehen.

### Beispiel 4: Destillative Trennung von MNT- und DNT-Isomeren

Es wurde ein Gemisch von 362 g MNT-Isomeren und 168 g DNT-Isomeren in einem 1 Liter Mehrhalskolben vorgelegt und unter Rühren über eine Laborkolonne (35 cm lange Vigreux-Kolonne) fraktionsweise destilliert. Es wurde ein Kopfdruck von 7,5 mbar eingestellt. Die Sumpftemperatur stieg kontinuierlich von 95 auf 145°C an. Parallel hierzu stieg die Kopftemperatur von 85 auf 98°C an. Die fraktionierten, akkumulierten Destillatzusammensetzungen wurden gaschromatographisch bestimmt zu:

Während die ersten 10 g Destillat DNT-frei waren, ließen sich erstmals nach 177,3 g Destillat DNT-Spuren nachweisen. Die letzte Destillatfraktion (nach 362,5 g) zeigte einen Gehalt von 5,4 Gew.% DNT. Die Endzusammensetzung des verbliebenen Sumpfes betrug bei einer Masse von 167,5 g: 0,6 Gew.% MNT und 99,4 Gew.% DNT.

## Patentansprüche

1. Verfahren zur Herstellung von Dinitrotoluolen durch adiabatische Nitrierung von Toluol mit Nitriersäure bei Temperaturen von 60 bis 200°C und einem Molverhältnis von Toluol zu Salpetersäure von 1:1,5 bis 1:3,0, **dadurch gekennzeichnet, daß** man das erhaltene Reaktionsgemisch bis zu einem Wassergehalt von bis zu 30 Gew.% aufkonzentriert, das im Reaktionsgemisch vorhandene Dinitrotoluol vor oder nach der Aufkonzentrierung aus dem Reaktionsgemisch ganz oder teilweise entfernt, das in den Brüden, die bei der Aufkonzentrierung des Reaktionsgemisches erhalten werden, noch vorhandene DNT durch Zugabe eines Lösungsmittels flüssig hält, das den Brüden zugegebene Lösungsmittel zusammen mit dem DNT von der wäßrigen Phase separiert, anschließend entweder das DNT von dem Lösungsmittel trennt, das Lösungsmittel wieder den Brüden zuführt und das aus den Brüden abgetrennte DNT entweder mit dem aus dem Reaktionsgemisch erhaltenen DNT vereinigt oder wieder in die Reaktion zurückführt oder das zusammen mit dem Lösungsmittel separierte DNT direkt in die Reaktion wieder zurückführt.

2. Verfahren nach Anspruch 1, **dadurch gekennzeichnet, daß** man die Nitrierung bei 90 bis 180°C durchführt.

3. Verfahren nach Ansprüchen 1 und 2, **dadurch gekennzeichnet, daß** das Molverhältnis von Toluol zu Salpetersäure 1:1,7 bis 1:2,5 beträgt.

4. Verfahren nach Ansprüchen 1 bis 3, **dadurch gekennzeichnet, daß** die Nitriersäure ein Gemisch aus Schwefel- und Salpetersäure ist und einen Salpetersäuregehalt von 0,5 bis 15 Gew.-% hat.

5. Verfahren nach Ansprüchen 1 bis 4, **dadurch gekennzeichnet, daß** als Lösungsmittel organische Verbindungen eingesetzt werden, die bei Normaldruck einen Siedepunkt von 80 bis 250°C besitzen.

6. Verfahren nach Ansprüchen 1 bis 5, **dadurch gekennzeichnet, daß** als Lösungsmittel Toluol und/oder Mononitrotoluole eingesetzt werden.

7. Verfahren nach Ansprüchen 1 bis 6, **dadurch gekennzeichnet, daß** das Gewichtsverhältnis der eingesetzten Lösungsmittel zu in den Brüden vorhandenem DNT 50:1 bis 1:10 beträgt.

## Claims

1. A process for producing dinitrotoluenes by the adiabatic nitration of toluene with nitrating acid at temperatures of 60 to 200°C and at a molar ratio of toluene to nitric acid of 1:1.5 to 1:3.0, **characterised in that** the reaction mixture obtained is concentrated to a water content of up to 30 % by weight, the dinitrotoluene which is present in the reaction mixture is removed from the reaction mixture before or after the concentration stage, the DNT which is still present in the vapours which are obtained on concentration of the reaction mixture is kept liquid by adding a solvent, the solvent which is added to the vapours is separated, together with the DNT, from the aqueous phase, subsequently either the DNT is separated from the solvent, the solvent is recycled to the vapours and the DNT which is separated from the vapours is either combined with the DNT obtained from the reaction mixture or is recycled to the reaction, or the DNT which is separated together with the solvent is recycled directly to the reaction.

2. A process according to claim 1, **characterised in that** nitration is conducted at 90 to 180°C.

3. A process according to claims 1 and 2, **characterised in that** the molar ratio of toluene to nitric acid is 1:1.7 to 1:2.5.

4. A process according to claims 1 to 3, **characterised in that** the nitrating acid is a mixture of sulphuric and nitric acids and has a nitric acid content of 0.5 to 15 % by weight.

5. A process according to claims 1 to 4, **characterised in that** organic compounds which have boiling points from 80 to 250°C under normal pressure are used as solvents.

6. A process according to claims 1 to 5, **characterised in that** toluene and/or mononitrotoluenes are used as solvents.

7. A process according to claims 1 to 6, **characterised in that** the ratio by weight of the solvent used to the DNT which is present in the vapours ranges from 50:1 to 1:10.

## Revendications

1. Procédé pour la préparation de dinitrotoluène par nitration adiabatique de toluène avec un mélange sulfonitrique à des températures de 60 à 200°C et avec un rapport molaire du toluène à l'acide nitrique de 1:1,5 à 1:3,0, **caractérisé en ce que** l'on concentre le mélange réactionnel obtenu à une teneur en eau jusqu'à 30 % en poids, on élimine totalement ou partiellement du mélange réactionnel le dinitrotoluène présent dans le mélange réactionnel avant ou après la concentration, on maintient le DNT encore présent dans les fumées, lesquelles sont obtenues lors de la concentration du mélange réactionnel, à l'état liquide par addition d'un solvant, on sépare le solvant ajouté aux fumées avec le DNT de la phase aqueuse, ensuite soit on sépare le DNT du solvant, on renvoie le solvant dans les fumées et on purifie le DNT séparé des fumées soit avec le DNT obtenu à partir du mélange réactionnel ; soit on le renvoie dans la réaction ; soit on renvoie directement dans la réaction le DNT séparé avec le solvant.

2. Procédé selon la revendication 1, **caractérisé en ce que** l'on réalise la nitration à de 90 à 180°C.

3. Procédé selon les revendications 1 et 2, **caractérisé en ce que** le rapport molaire du toluène à l'acide nitrique est compris entre 1:1,7 et 1:2,5.

4. Procédé selon les revendications 1 à 3, **caractérisé en ce que** le mélange sulfonitrique est un mélange d'acide sulfurique et d'acide nitrique et présente une teneur en acide nitrique de 0,5 à 15 % en poids.

5. Procédé selon les revendications 1 à 4, **caractérisé en ce que** l'on utilise comme solvant des composés organiques qui présentent un point d'ébullition de 80 à 250°C à pression atmosphérique.

6. Procédé selon les revendications 1 à 5, **caractérisé en ce que** l'on utilise comme solvant du toluène et/ou du mononitrotoluène.

7. Procédé selon les revendications 1 à 6, **caractérisé en ce que** le rapport massique du solvant utilisé au DNT présent dans les fumées est compris entre 50:1 et 1:10.
